# EUROPEAN PATENT APPLICATION

(11) **EP 1 900 322 A1**
(43) Date of publication of application: **19.03.2008**
(21) Application number: 07116196.2
(22) Date of filing: 12.09.2007
(51) Int. Cl.: A61B 5/00, A61B 1/05

(54) **Device and method for displaying images received from an in-vivo imaging device**

(30) Priority: 14.09.2006 US 520681
(71) Applicant: Given Imaging Ltd., 20692 Yoqneam (IL)
(72) Inventor: Gilad, Zvika, 34987 Haifa (IL)
(74) Representative: Graf, Werner

(57) **Abstract**

A device and method for displaying images of the gastrointestinal tract, or other body lumens or cavities of a patient, captured by an imager of an in-vivo imaging device. Images taken using white light illumination may be compared with images taken using narrow band illumination by operating a display button.

## Description

### FIELD OF THE INVENTION

The present invention relates to the displaying of images received from an in-vivo imaging device. More specifically, the present invention relates to the displaying of two or more of such images side by side on single display screen.

### BACKGROUND OF THE INVENTION

In-vivo sensing devices for diagnosis of the gastrointestinal (GI) tract or other body lumens of a patient such as, for example, ingestible imaging capsules, may wirelessly transmit image data to an external data recorder. The data recorder may be affixed to the patient by a strap or a belt so that the patient may freely perform normal actions during an observation period that may begin after swallowing of the in-vivo imaging device and end upon its excretion. The data recorder may have radio communication capability and it may have connected to it one or more antennas for receiving the image data transmitted by the in-vivo imaging device and the data recorder may have a memory for storing the received image data. After the observation period, the patient may deliver the data recorder to an operator, for example, a health professional who may download the stored image data for processing and for performing analysis of the GI tract for diagnosis purposes.

The image data includes images of the GI tract captured by an imager in the in-vivo imaging device as it passes through the GI tract. The image data may be downloaded from the data recorder to a workstation, or the like, where it may undergo various forms of image processing prior to analysis of the images of the GI tract for diagnosis purposes. The images may be obtained using broad spectrum white light illumination, or narrow band illumination of a given spectral range, depending on the information required. In certain circumstances, in order to augment the analysis, it may be desirable to compare the images obtained using white illumination with those obtained using narrow band illumination.

### SUMMARY OF THE INVENTION

According to embodiments of the present invention, there is provided a display device for displaying images captured by an imager of an in-vivo imaging device, the display device comprising:
a display unit including a display screen, for displaying the captured images;
a white light illumination display button, for displaying images captured using white light illumination; and
a narrow band illumination display button, for displaying images captured using narrow band illumination of a given spectral range.

According to some embodiments, the display device comprises a data processor storage unit for storing the captured images and a data processor for processing the stored images.

According to some embodiments, the white light and narrow band illumination display buttons are software buttons.

According to embodiments of the present invention, there is also provided a method for displaying images captured by an imager of an in-vivo imaging device, the method comprising:
receiving image data from the in-vivo imaging device, the image data comprising image frames captured under different illumination conditions;
displaying on a screen of a display unit at least one first image frame captured under a first illumination condition; and
displaying on the screen at least one second image frame captured under a second illumination condition along with the at least one first image frame.

According to some embodiments, the first illumination condition consists of illuminating with white light a target area from which the image frames are captured.

According to some embodiments, the second illumination condition consists of illuminating with narrow band illumination of a given spectral range the target area from which the image frames are captured.

According to some embodiments, the first and second illumination conditions consist of illuminating with narrow band illumination of given spectral ranges the target area from which the image frames are captured, the given spectral range for the first illumination condition being different from the given spectral range for the second illumination condition.

In accordance with some embodiments, the given spectral range is selected from the group consisting of: ultraviolet radiation, infrared radiation, blue light and red light

According to some embodiments, the at least one first and second image frames are displayed adjacent each other.

In accordance with some embodiments, the at least one first image frame is from a first image data stream of the received image data captured under the first illumination condition.

In accordance with some embodiments, the at least one second image frame is from a second image data stream of the received image data captured under the second illumination condition.

In accordance with some embodiments, the method comprises dividing the received image data into image data streams, each image data stream corresponding to image frames captured under a given illumination condition.

### BRIEF DESCRIPTION OF THE DRAWINGS

The present invention will be understood and appreciated more fully from the following detailed description taken in conjunction with the drawings in which:
Fig. 1 is a simplified conceptual illustration of an in-vivo imaging system according embodiments of the present invention;
Fig. 2 is an illustrative end view of the in-vivo imaging device shown in Fig. 1 in accordance with embodiments of the present invention;
Fig. 3 is a simplified illustrative side view of an in-vivo imaging device with illumination sources at both ends;
Fig. 4 is an illustrative end view of the in-vivo imaging device of Fig. 3 in accordance with embodiments of the present invention;
Fig. 5 is an illustrative diagram of a display unit displaying a first image frame captured under a first illumination condition in accordance with some embodiments of the present invention;
Fig. 6 is an illustrative diagram of a display unit displaying a first image frame captured under a first illumination condition and a second image frame captured under a second illumination condition in accordance with some embodiments of the present invention; and

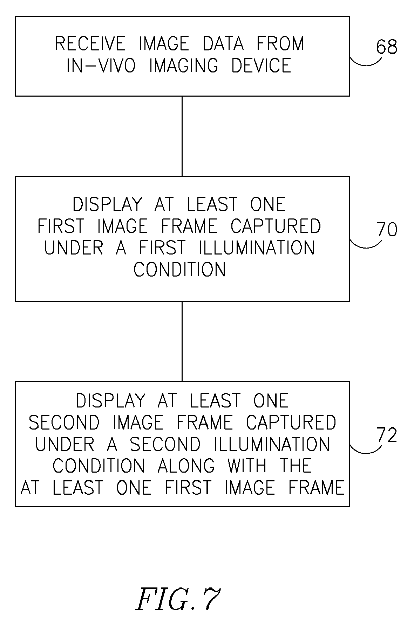

Fig. 7 is a flowchart depicting a method according to an embodiment of the invention.

It will be appreciated that for simplicity and clarity of illustration, elements shown in the figures have not necessarily been drawn accurately or to scale. For example, the dimensions of some of the elements may be exaggerated relative to other elements for clarity, or several physical components may be included in one functional block or element. Further, where considered appropriate, reference numerals may be repeated among the figures to indicate corresponding or analogous elements.

### DETAILED DESCRIPTION OF THE INVENTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well-known features may be omitted or simplified in order not to obscure the present invention.

The device and method of the present invention may be used with an imaging system or device such as that described in U.S. Patent No. 5,604,531 entitled "In Vivo Video Camera System," which is incorporated herein by reference. A further example of an imaging system and device with which the device and method of the present invention may be used is described in U.S. Patent No. 7,009,634 entitled "Device for In Vivo Imaging," which is incorporated herein by reference. For example, a swallowable imaging capsule such as that described in U.S. Patent No. 7,009,634, may be used in the present invention. A further example of swallowable imaging capsules that may be used with the device and method of the present invention are those described in U.S. Patent Application Publication No. 2002/0109774 entitled "System and Method Wide Field Imaging of Body Lumens," which is incorporated herein by reference.

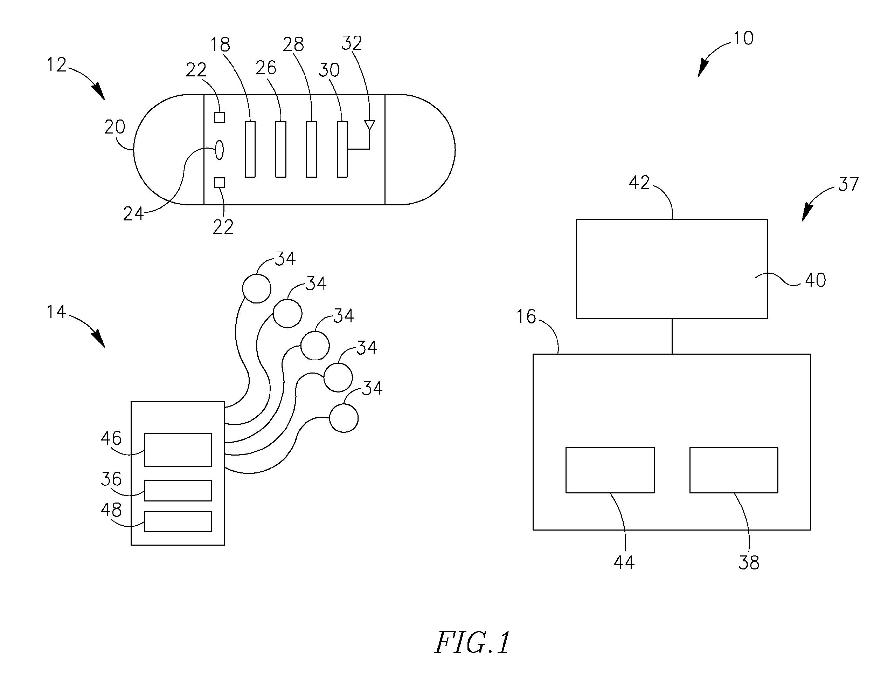

Reference is made to Fig. 1, showing in-vivo imaging system 10 according to embodiments of the present invention. The in-vivo imaging system 10 includes an in-vivo imaging device 12, a data recorder 14 and a work station 16. In some embodiments, the in-vivo imaging device 12 may be a wireless device. In some embodiment, the in-vivo imaging device 12 may be autonomous. In some embodiments, the in-vivo imaging device 12 may be a swallowable capsule for imaging the gastrointestinal tract of a patient. However, other body lumens or cavities may be imaged or examined with the in-vivo imaging device 12.

The in-vivo imaging device 12 may be cylindrical in shape with dome-like ends and may include at least one imager 18 for capturing image data in the form of image frames of images of the gastrointestinal tract or other body lumens or cavities, a viewing window 20 at one of the ends, one or more illumination sources 22, an optical system 24, a power supply such as a battery 26, a processor 28, a transmitter 30, and an antenna 32 connected to the transmitter 30. As the in-vivo imaging device 12 traverses the gastrointestinal tract or other body lumens of a patient, it takes a series of images thereof. The images may be taken at a given rate, i.e., a given number of frames per second. The series of images captured by the imager 18 of the in-vivo imaging device 12 may form a data stream, forming the frames of a video movie. The imager 18 may be and/or contain a CMOS imager. Alternatively, other imagers may be used, e.g. a CCD imager or other imagers.

The image data and or other data captured by the in-vivo imaging device 12 may be transmitted as a data signal by wireless connection, e.g. by wireless communication channel, by the transmitter 30 via the antenna 32, from the in-vivo imaging device 12 and received by the data recorder 14 via one or more receiving antennas 34, for example an antenna array that may, for example, at least partially surround the patient. The received data signal may be stored on a data recorder storage unit 36. The received data signal may be, for example, downloaded to the workstation 16 of a display device 37 for processing by a work station processor 38, and for analysis, and display, for example, on a display screen 40 of a display unit 42 of the display device 37. The received processed data may be stored on a workstation storage unit 44. Downloading and/or processing in the workstation 36 may occur off-line for example after the data recorder 14 has completed receiving and recording the data signal received from the in-vivo imaging device 12, or may occur in real-time. In one embodiment of the present invention, the data recorder 14 and the workstation 16 may be integrated into a single unit, for example, may be integrated into a single portable unit. In yet another embodiment of the present invention, the data recorder 14 may include display capability, for example the data recorder 14 may include a viewer 46 for viewing information and/or images, for example information and/or images transmitted by the in-vivo imaging device 12. In another embodiment, processing and/or analysis may be performed at least partially within the data recorder 14 by a data recorder processor 48.

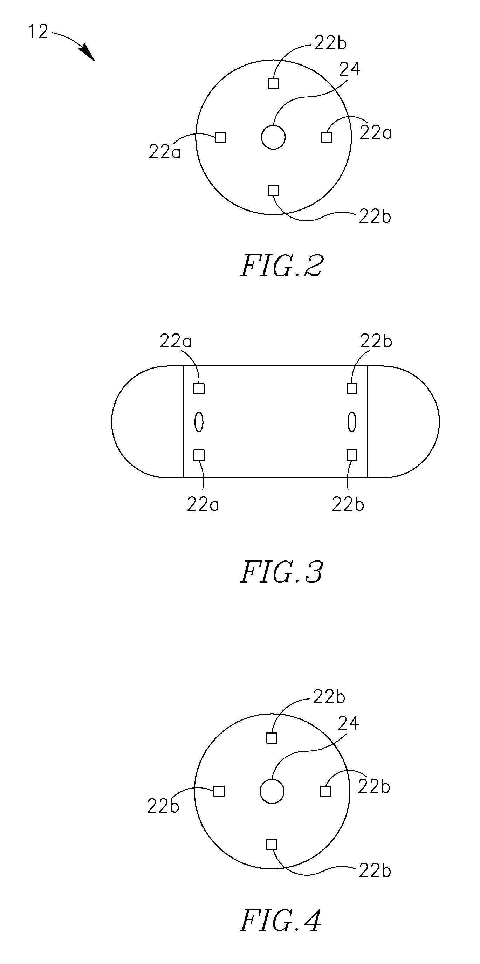

Reference is now made to Fig. 2. The illumination sources 22 (22a, 22b) may be Light Emitting Diodes (LED) and may be located at one end the in-vivo imaging device 12, for illuminating a target area from which image frames are to be captured. The target area may be an area of the gastrointestinal tract or other body lumens or cavities of the patient. In accordance with some embodiments, some of the illumination sources 22 may be white light illumination sources 22a and some may be narrow band illumination sources 22b. The white light and narrow band illumination sources 22a, 22b may be operated alternately at given time intervals. Consequently, the data stream of captured image frames may contain both image frames captured under both white light and narrow band illumination conditions. In accordance with some embodiments, the data stream of captured image frames may be divided into separate image data streams, each image data stream corresponding to image frames captured under a given illumination condition. The dividing of the data stream of captured image frames into separate image data streams for each illumination condition may be performed in the workstation 16, for example, by the work station processor 38.

In accordance with some embodiments, the narrow band illumination sources 22b may be of a given spectral range thereby giving rise to a given narrow band illumination of a specific type of radiation, or color of light. In accordance with some embodiments, some of the narrow band illumination sources 22b may illuminate with ultraviolet radiation or infrared radiation. The word illuminate is used in the specification and claims not just for visible radiation but in a generalized manner to include radiation which is not in the visible region of the optical spectrum. In accordance with some embodiments, some of the narrow band illumination sources 22b may illuminate with blue light or red light.

In accordance with a specific embodiment, the in-vivo imaging device 12 may include four illumination sources 22 at one of its ends, two of the illumination sources may be white light illumination sources 22a and the other two illumination sources may be narrow band illumination sources 22b. In some embodiments, the two narrow band illumination sources 22b may be narrow band illumination sources with the same narrow spectral range. For example, the two narrow band illumination sources 22b may both be sources of infra-red radiation, or both be sources or ultra-violet radiation, or both be sources of red light radiation, or both be sources of blue light radiation. In some embodiments, the two narrow band illumination sources 22b may have different narrow spectral ranges. For example, one of the two narrow band illumination sources 22b may be a source of ultraviolet radiation whilst the other may be a source of either infrared radiation, or blue light radiation or red light radiation.

Attention is now drawn to Figs. 3 and 4. According to some embodiments the in-vivo imaging device 12 may have illumination sources at both of its ends, allowing it to capture images in both a forward and rearward direction, relative to the direction of motion, as it traverses the gastrointestinal tract or other body lumens of a patient. The illumination sources at one end of the in-vivo imaging device 12 may be white light illumination sources 22a and the illumination sources at the other one end may be narrow band illumination sources 22b. In some embodiments, the narrow band illumination sources 22b may be narrow band illumination sources with the same narrow spectral range. For example, the narrow band illumination sources 22b may all be sources of infra-red radiation, or all be sources or ultra-violet radiation, or all be sources of red light radiation, or all be sources of blue light radiation. In some embodiments, the narrow band illumination sources 22b may have different narrow spectral ranges. For example, some of the narrow band illumination sources 22b may be a source of ultraviolet radiation whilst the others may be a source of either infrared radiation, or blue light radiation or red light radiation. Other combinations are possible, for example, some of the narrow band illumination sources 22b may be a source blue light radiation whilst the others may be a source of red light radiation.

According to other embodiments imaging device 12 may have other shapes and the illumination sources need not be located at an end of the device, rather they may illuminate through a side window or other window.

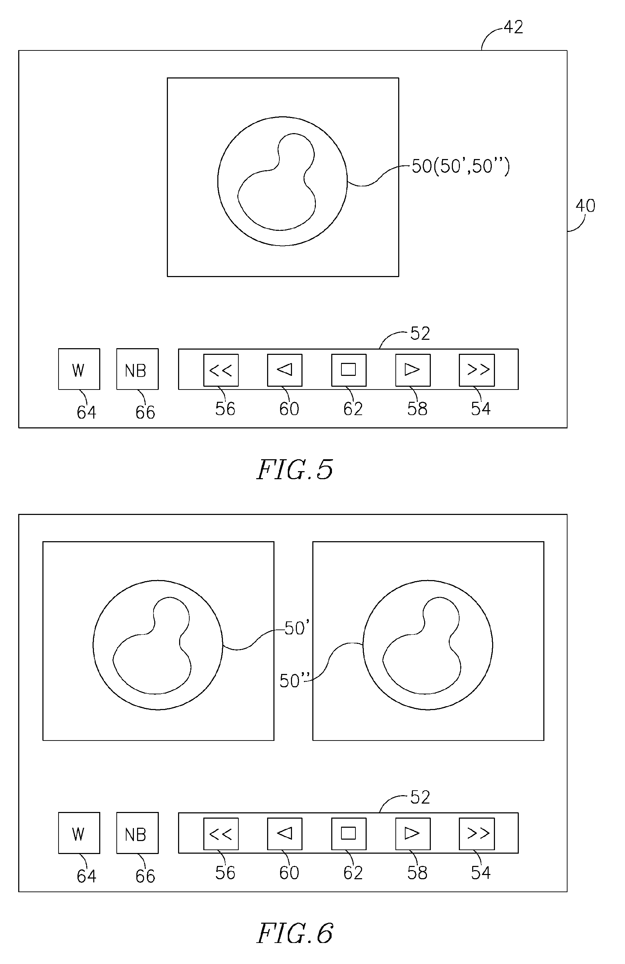

Reference is now made to Fig. 5 showing images 50 displayed on the display screen 40 of the display unit 42 in accordance with some embodiments of the invention. The displayed images 50 may either be single still images (i.e., single image frames) or a movie of the data stream of images captured by the imager 18 of the in-vivo imaging device 12. Also shown in Fig. 5 is a set of control buttons 52 for controlling the viewing of the video movie. The set of control buttons 52 may be a set of software buttons appearing on the display screen 40. In accordance with some embodiments, the set of control buttons 52 may include a fast forward control button 54, a fast backward (or rewind) control button 56, a play control button 58, and reverse play control button 60 and a stop control button 62. In accordance with some embodiments, illumination display buttons, a white light illumination display button 64 and a narrow band illumination display button 66 may also be provided. The illumination display buttons 64, 66 may be software buttons appearing on the display screen 40.

According to some embodiments, upon operating the play control button 58 (e.g., by using a mouse to locate the cursor on the display screen 40 over the play control button 58 and left clicking the mouse) there is displayed on the display screen 40 at least one first image frame 50' captured under a first illumination condition. The at least one first image frame 50' may be an image frame of a first movie of the data stream of images captured under a first illumination condition.

Attention is now drawn to Fig. 6. According to some embodiments, by operating one of the illumination display buttons 64, 66 there is displayed on the display screen 40 at least one second image frame 50" captured under a second illumination condition, in addition to the at least one first image frame 50' captured under a first illumination condition. According to some embodiments, the first illumination condition consists of illuminating with white light a target area from which the image frames are captured. According to some embodiments, the second illumination condition consists of illuminating with narrow band illumination of a given spectral range the target area from which the image frames are captured. According to some embodiments, the first and second illumination conditions consist of illuminating with narrow band illumination of given spectral ranges the target area from which the image frames are captured, the given spectral range for the first illumination condition being different from the given spectral range for the second illumination condition.

In accordance with some embodiments, the given spectral range of the narrow band illumination may be selected from the group consisting of: ultraviolet radiation, infrared radiation, blue light and red light.

Accordingly, an operator, such as a health professional, may inspect, on the display screen 40, a video movie of the data stream of images captured by the imager 18 of the in-vivo imaging device 12 as it traverses the gastrointestinal tract or other body lumens of a patient, taken using white light illumination. The operator may desire to compare the image of a certain target area taken using white light illumination with an image of the same target area taken using narrow band illumination. In accordance with embodiments of the present invention, upon operating the narrow band illumination display button 66, the required image of the same target area taken using narrow band illumination appears alongside the image of the same target area taken using white light. According to some embodiments, a video movie of the gastrointestinal tract or other body lumens of a patient taken using narrow band illumination may be displayed on the display screen 40 and comparison may be made with a video movie (or frames of a specific target area) taken using white light illumination by operating the white light illumination display button 64 thereby displaying video movie (or frames of the specific target area) taken using white light illumination alongside the video movie taken (or frames of the specific target area) using narrow band illumination.

The present invention also provides a method for displaying images 50 captured by an imager 18 of an in-vivo imaging device 12. In accordance with some embodiments, image data may be received from the in-vivo imaging device (box 68). The image data may comprise image frames captured under different illumination conditions. At least one first image frame captured under a first illumination condition may be displayed on the display screen 40 of the display unit 42 (box 70); and at least one second image frame captured under a second illumination condition may be displayed on the display screen 40 of the display unit 42 (box 72). The at least one second image frame may be displayed by operating a specific illumination display button 64,66.

When viewing a video movie of the data stream of images captured by the imager 18 of the in-vivo imaging device 12 as it traverses the gastrointestinal tract or other body lumens of a patient, for example for medical diagnosis, the viewer may desire to record annotations regarding certain portions or frames, or may wish to "bookmark" certain portions or frames, as, for example, described in U.S. Patent Application Publication No. 2002/0171669 entitled "System and method for annotation on a moving image," which is incorporated herein by reference.

In an exemplary embodiment, the user may annotate portions of the movie. When used herein, a "portion" of a movie may include a single still image, a set of still images, or a series of still images which may be displayed as a moving image. When used herein, "annotation" and its derivatives may indicate comments or any additional item of information or data added to or linked to a moving image or a portion of the movie. For example, an annotation may include, but is not limited to, a textual, audio, or other note which is associated with a portion of the movie, a bookmark, tab or label which is associated with a portion of the movie, or a medical diagnosis or description of the portion. Annotations may be stored in the work station storage unit 44.

A bookmark may be, for example, an indication or marker, or an index entry, which indicates to a user a portion of the moving image which is of interest. For example, a user may bookmark a frame which depicts a pathology in a GI tract. An annotation may be useful in a system where a large number of image frames are stored, only a certain number of which are relevant to a diagnosis. The user may store the image sequence and use the annotations to find the relevant portions and to record significant facts about those portions. When wishing to find portions of the movie which are of interest, the user, or other users, may refer to the bookmarks or annotations.

In an exemplary embodiment, an annotation includes or refers to sets of images (which may include only one image), a time marking the time elapsed for the image (or the earliest of the set of images), and text. In an alternate embodiment the time may be replaced with another suitable measure, such as frames elapsed, etc. Each image may be either an actual image, stored in a known format, or may be a link to an image in the image file. In alternate embodiments, an annotation may include other combinations of information, including, for example, data in a non-textual format. Preferably, the annotations associated with a movie may be exported from the system or saved as a file, and reports summarizing or otherwise organizing information in the annotations may be generated.

In some embodiments, a timeline window on the display screen 40 may provide a timeline, an indication of the total time elapsed for the movie, and may provide other information, such as the total time of the movie. In an exemplary embodiment, the timeline may be a bar labeled with time units, having summaries of annotations visually attached to the timeline at portions which indicate the time elapsed for the portions associated with the annotations.

The annotations and/or bookmarks and the timeline window may be applied separately to movies obtained under both white light and narrow band illumination conditions.

In addition to the control buttons shown in Figs. 5 and 6, there may also be buttons assigned to assessing a patient's condition. For example, for blood detection or for detecting the presence of inflammatory diseases such as ulcerative colitis and Crohn's disease. Such buttons may be used to assign a weight to parameter values, typically parameters which define mucosal damage, for computing a score or number for selected images in order to enable assessment of changes in the condition of a patient's gastrointestinal tract.

While the present invention has been described with reference to one or more specific embodiments, the description is intended to be illustrative as a whole and is not to be construed as limiting the invention to the embodiments shown. It is appreciated that various modifications may occur to those skilled in the art that, while not specifically shown herein, are nevertheless within the scope of the invention.

## Claims

1. A display device for displaying images captured by an imager of an in-vivo imaging device, the display device comprising:
a display unit including a display screen, for displaying the captured images;
a white light illumination display button, for displaying images captured using white light illumination; and
a narrow band illumination display button, for displaying images captured using narrow band illumination of a given spectral range.

2. The display device according to claim 1, comprising a data processor storage unit for storing the captured images and a data processor for processing the stored images.

3. The display device according to claim 1 or 2, wherein the white light and narrow band illumination display buttons are software buttons.

4. A method for displaying images captured by an imager of an in-vivo imaging device, the method comprising:
receiving image data from the in-vivo imaging device, the image data comprising image frames captured under different illumination conditions;
displaying on a screen of a display unit at least one first image frame captured under a first illumination condition; and
displaying on the screen at least one second image frame captured under a second illumination condition along with the at least one first image frame.

5. The method according to claim 4, wherein the first illumination condition consists of illuminating with white light a target area from which the image frames are captured.

6. The method according to claim 4 or 5, wherein the second illumination condition consists of illuminating with narrow band illumination of a given spectral range the target area from which the image frames are captured.

7. The method according to one of claims 4 to 6, wherein the first and second illumination conditions consists of illuminating with narrow band illumination of given spectral ranges the target area from which the image frames are captured, the given spectral range for the first illumination condition being different from the given spectral range for the second illumination condition.

8. The method according to claims 6 and 7, wherein the given spectral range is selected from the group consisting of: ultraviolet radiation, infrared radiation, blue light and red light.

9. The method according to one of claims 4 to 8, wherein the at least one first and second image frames are displayed adjacent each other.

10. The method according to one of claims 4 to 9, wherein the at least one first image frame is from a first image data stream of the received image data captured under the first illumination condition.

11. The method according to claim 10, wherein the at least one second image frame is from a second image data stream of the received image data captured under the second illumination condition.

12. The method according to one of claims 4 to 11, comprising dividing the received image data into image data streams, each image data stream corresponding to image frames captured under a given illumination condition.
